# EUROPÄISCHE PATENTANMELDUNG

(11) **EP 0 775 517 A1**
(43) Veröffentlichungstag der Anmeldung: **28.05.1997**
(21) Anmeldenummer: 96116618.8
(22) Anmeldetag: 17.10.1996
(51) Int. Cl.: B01F 17/00

(54) **Wasser/Öl-Mischemulgator sowie dessen Verwendung in kosmetischen und pharmazeutischen Zubereitungen**

(30) Priorität: 24.10.1995 DE 19539429
(71) Anmelder: Kawes S.L., 08016 Barcelona (ES)
(72) Erfinder: Engel, Walter, 25421 Pinneberg (DE)
(74) Vertreter: Freiherr von Uexküll, Jürgen-Detlev, Dr. Rer. Nat. Dipl.-Chem.

(57) **Zusammenfassung**

Die Erfindung betrifft einen Wasser/Öl-Misch-Emulgator mit einem Gehalt an C-10 bis C-28 Fettalkoholen sowie dessen Verwendung in kosmetischen und pharmazeutischen Zubereitungen, wobei der Emulgator
a) 6 bis 13 Gew.% Phytosterole und
b) 5 bis 23 Gew.% C-20 bis C-24 2-Alkylalkanole enthält, wobei
c) der Anteil aus C-10 bis 28 n-Fettalkoholen die folgende Zusammensetzung hat:

| | |
|---|---|
| C-10: | 0,0 bis 1,0 Gew.% |
| C-12: | 1,0 bis 3,0 Gew.% |
| C-14: | 12,0 bis 16,0 Gew.% |
| C-16: | 16,0 bis 21,0 Gew.% |
| C-18: | 11,0 bis 14,5 Gew.% |
| C-20: | 6,0 bis 10,0 Gew.% |
| C-22: | 7,0 bis 11,0 Gew.% |
| C-24: | 2,5 bis 5,0 Gew.% |
| C-26: | 0,5 bis 2,5 Gew.% |
| C-28: | 0,5 bis 1,1 Gew.%. |

## Beschreibung

Die vorliegende Erfindung betrifft einen Wasser/Öl-Misch-Emulgator mit einem Gehalt an C-10 bis C-28 Fettalkohol sowie dessen Verwendung in kosmetischen und pharmazeutischen Zubereitungen.

Wasser/Öl-Emulgatoren auf Basis von Lanolin bzw. Wollwachsalkoholen sind bekannt und enthalten neben Steroiden und Triterpenalkoholen geradkettige und verzweigtkettige C-12 bis C-28 Alkohole. Die Aufarbeitung der aus dem Wollfett stammenden Emulgatoren ist überaus aufwendig, darüber hinaus ist bei den Wollwachsalkoholen bzw. dem Lanolin ein allergenes Potential nicht auszuschließen. Ferner ist die Qualität dieser Produkte aufgrund der unterschiedlichen Herkunft des tierischen Materials nicht konstant. Andere Wasser/Öl-Emulgatoren auf Basis von Pentaerytheritfettsäureester, Salze höherer Fettsäuren, Sorbitanfettsäureester, Phytosterinen, Cholesterin und anderen ergeben Stabilitätsprobleme bei der Verwendung dieser Wasser/Öl-Emulgatoren.

Die Verwendung von Wasser/Öl-Emulgatoren zur Herstellung von kosmetischen und pharmazeutischen Zubereitungen wie Salben, Cremes, Lotionen als alleiniger oder zusätzlicher Emulgator und auch als Rückfettungskomponente in kosmetischen und pharmazeutischen Reinigungsmitteln wie Seifen, Shampoos und Badelotionen ist an sich bekannt, wobei beispielsweise nach dem deutschen Arzneibuch 3,0 Gew.% Wollwachsalkohole und 0,25 Gew.% Cetylstearylalkohol mit etwa 46 Gew.% Vaseline und 50 Gew.% Wasser zu einer wasserhaltigen Salbe verarbeitet werden können.

Gemäß DE 26 23 927 C2 werden als Emulgatoren für kosmetische Emulsionen Phytosterine, die jedoch einen relativ hohen Schmelzpunkt von etwa 136 bis 140° C haben, vorgeschlagen; um diese leichter zu Emulsionen zu verarbeiten, wurde hier vorgeschlagen, diese Phytosterine in einer Menge von 10 bis 50 Gew. % mit 50 bis 90 Gew. % eines einzigen freien gesättigten C-12 bis C-18 Alkohols zu vermischen. Als Alkohol wurde einmal Dodecylalkohol oder Cetylalkohol oder Myristylalkohol oder Stearylalkohol vorgeschlagen. Hierbei wurde zwar die Einarbeitung der getrennten Bestandteile Phytosterin einerseits und Stearylalkohol andererseits und auch die spätere Verarbeitung mit einem Mineralöl erleichtert, jedoch waren sowohl der Gehalt an den kostspieligen Phytosterinen zu hoch als auch die Emulgiereigenschaften nicht ausreichend.

Gemäß DE 41 25 332 A1 wurde zur Herstellung eines klaren flüssigen Emulgatorkonzentrats vorgeschlagen, eine geringe Menge von 7 bis 15 Gew.% von Sterinen oder Wollwachsalkoholen mit einer überwiegenden Menge von 85 bis 93 Gew.% eines verzweigten, gesättigten C-16 bis C-20 Alkohols, nämlich eines hochverzweigten 2-Hexyldecanols oder 2-Octyldodecanols einzusetzen.

Auch bei diesen Emulgatorkonzentraten lag der Anteil an Wollwachsalkohol bzw. Sterol oder Sterinen bezogen auf eine Standardrezeptur verhältnismäßig hoch, was zu kostenungünstigen bzw. unwirtschaftlichen Endprodukten führte.

Aufgabe der vorliegenden Erfindung ist es, einen neuen Wasser/Öl-Misch-Emulgator vorzuschlagen, der in stets gleichbleibender Reinheit, Emulgierkraft und Emulsionsstabilität den Naturprodukten wie Lanolin überlegen ist und insbesondere frei von tierischen und mineralischen Fetten und Ölen sowie von Wachsestern wie z.B. Lanolin ist, der andererseits ein nichtionischer und EO-freier Emulgator ist und die Eigenschaft von Lanolinalkohol-Emulgatoren mit niedrigem Schmelzbereich und guter Verarbeitbarkeit zeigt.

Zur Lösung dieser Aufgabe wird ein Wasser/Öl-Misch-Emulgator gemäß Hauptanspruch vorgeschlagen, wobei weitere bevorzugte Ausführungsformen bzw. dessen Verwendung in den Unteransprüchen definiert sind.

Überraschenderweise wurde gefunden, daß ein Anteil von 6 bis zu 13 Gew.% Phytosterolen und von 15 bis 23 Gew.% C-20 bis C 24 2-Alkylalkanolen in Kombination mit einer Mischung aus C-10 bis C-28 n-Fettalkoholen eine erhebliche Steigerung der Emulgatoreigenschaften bewirkt.

Eine Abmischung aus üblichen im Handel erhältlichen kettenreinen C-10 bis C-30 Fettalkoholen zeigt zwar hinreichende Emulgatoreigenschaften, die aber bei weitem nicht an die von Wollwachsalkoholen heranreicht. Werden allerdings die C-24 bis C-26 n-Alkohole teilweise durch C-20 bis C-24 Isoalkohole, d.h. 2-Alkylalkanole, ersetzt, wird die Emulgatoreignung signifikant verbessert, wodurch auch der Anteil an Phytosterolen bzw. Phytosterinen verringert werden kann.

Im folgenden soll die Erfindung anhand von Beispielen näher erläutert werden:

### Beispiel 1:

Es wurde eine Mischung C-10 bis C-28 Fettalkoholen der folgenden Zusammensetzung zubereitet:

| | |
|---|---|
| C-10: | 0,1 Gew.%, |
| C-12: | 1,6 Gew.%, |
| C-14: | 14,1 Gew.%, |
| C-16: | 18,2 Gew.%, |
| C-18: | 13,1 Gew.%, |
| C-20: | 8,1 Gew.%, |
| C-22: | 9,4 Gew.%, |
| C-24: | 3,5 Gew.%, |
| C-26: | 1,5 Gew.%, |
| C-28: | 0,8 Gew.%. |

jeweils bezogen auf die Gesamt-Zusammensetzung des Wasser/Öl-Misch-Emulgators.

Zu dieser C-10 bis C-28-n-Fettalkoholkomponente wurden insgesamt 19,8 Gew.% C-20 bis C-24 2-Alkylalkanol, und zwar 2,8 Gew.% C-20 und 17 Gew.% C-24 iso-Alkanol zugegeben, während der Rest durch Phytosterole ergänzt wurde. Diese Phytosterole bestanden im vorliegenden Fall aus etwa 60 % Sitosterin, 34 % Campesterin und 6 % Stigmasterin.

### Beispiel 2:

Der gemäß Beispiel 1 erhaltene Wasser/Öl-Misch-Emulgator wurde zu einer Creme verarbeitet, die die folgende Zusammensetzung hatte:

| | |
|---|---|
| Emulgator gemäß Beispiel 1: | 2,3 Gew.% |
| Paraffinöl: | 4,2 Gew.% |
| Vaseline: | 27,8 Gew.% |
| Wasser: | 65,7 Gew.%. |

Die mit dem erfindungsgemäßen Emulgator hergestellte Creme war eine weiße, schön glänzende, geschmeidige Zubereitung mit einer Lagerstabilität zwischen 4° bis 45° C. Das Wasseraufnahmevermögen lag bei etwa 65 %.

### Vergleichsversuch:

Es wurde anstelle des erfindungsgemäßen Emulgators ein Wollwachsalkohol zusammen mit einem Cetylstearylalkohol entsprechend DAB mit den folgenden Mengen verarbeitet:

| | |
|---|---|
| Wollwachsalkohole: | 3,0 Gew.% |
| Cetylstearylalkohol: | 0,25 Gew.% |
| Vaseline: | 46,75 Gew.% |
| Wasser: | 50 Gew.% |

Die mit diesem bislang am besten beurteilten Emulgator verarbeitete Salbe zeigte einen geringeren Glanz und war verarbeitungstechnisch nachteilig, weil sehr viel mehr Vaseline, nämlich 46,75 % gegenüber 27,8 % eingesetzt werden mußte und das Wasseraufnahmevermögen nur bei 50 % gegenüber 65,7 % geringer war.

### Beispiel 3:

Es wurde ein Wasser/Öl-Misch-Emulgator analog Beispiel 1 hergestellt, wobei jedoch jetzt der Anteil der Isofettalkohole bei 3,0 Gew.% C-20-2-Alkylalkanol und 16,8 Gew.% C-24-2-Alkylalkanol lag.

Die Ergebnisse einer mit diesem Emulgator erzielten Creme analog Beispiel 2 waren nahezu gleich, jedoch zeigte sich eine noch bessere, weiß glänzende Struktur.

### Beispiel 4:

Es wurde eine W/O-Emulsion der folgenden Zusammensetzung hergestellt:

| | |
|---|---|
| Emulgator gemäß Beispiel 1: | 2,00 Gew.% |
| Mg-Stearat: | 1,50 Gew.% |
| Al-Stearat: | 0,75 Gew.% |
| weißes Bienenwachs: | 3,00 Gew.% |
| Vaseline, weiß (DAB): | 5,00 Gew.% |
| Paraffinöl, dickfl. (DAB): | 15,00 Gew.% |
| Isopropylmyristat: | 5,00 Gew.% |
| Butylhydroxytoluol: | 0,01 Gew.% |
| Glyzerin: | 3,00 Gew.% |
| Magnesiumsulfat: | 0,70 Gew.% |
| Wasser: | 63,00 Gew.% |
| Konservierungsmittel, Parfum: | 1,04 Gew.% |

Diese Emulsion war von hoher kosmetischer Anmutung.

### Beispiel 5:

Es wurde eine Pflegecreme der folgenden Zusammensetzung hergestellt:

| | |
|---|---|
| Emulgator gemäß Beispiel 2: | 3,40 Gew.% |
| weißes Bienenwachs: | 1,00 Gew.% |
| Vaseline, weiß (DAB): | 3,00 Gew.% |
| Mikrowachs: | 1,00 Gew.% |
| Paraffinöl, dickfl. (DAB): | 3,00 Gew.% |
| Isopropylmyristat: | 10,00 Gew.% |
| Cetearyl-Isononanoat "Cetiol SN": | 8,00 Gew.% |
| Glyzerin: | 4,00 Gew.% |
| Magnesiumsulfat: | 0,70 Gew.% |
| Wasser: | 64,00 Gew.% |
| Konservierungsmittel, Parfum: | 1,90 Gew.% |

Diese Pflegecreme zeigte besonders gute Pflegeeigenschaften und kann beispielsweise als Nachtcreme verwendet werden.

### Beispiel 6:

Es wurde eine Toiletteseife mit überfettenden Eigenschaften der folgenden Zusammensetzung hergestellt:

| | |
|---|---|
| Grundseife: | 98,29 Gew.% |
| Emulgator gemäß Beispiel 1: | 0,39 Gew.% |
| Farbstoff: | 0,01 Gew.% |
| Antioxydans: | 0,05 Gew.% |
| Parfum: | 1,07 Gew.% |
| Titandioxyd: | 0,19 Gew.% |

Die Grundseife bestand aus einer Natriumkernseife mit etwa 20 % Wasser und einem Gehalt von etwa 78 % Talg und Kokosfett-Fettsäuren.

Diese Seife zeichnete sich insbesondere durch einen besonders feinen Schaum und ein ausgezeichnetes Hautgefühl aus.

### Beispiel 7:

Es wurde ein weicher Schaumbad-Zusatz (Tubenware) der folgenden Zusammensetzung hergestellt:

| | |
|---|---|
| Lauryläthersulfat (70 %ig): | 40,00 Gew.% |
| Ölsäurediäthanolamid: | 4,00 Gew.% |
| Aethylenglykolstearat: | 4,00 Gew.% |
| Emulgator gem. Beispiel 1: | 0,50 Gew.% |
| Isopropylmyristat: | 5,00 Gew.% |
| Natriumchlorid: | 5,00 Gew.% |
| Wasser: | 40,00 Gew.% |
| Konservierungsmittel, Parfum: | 1,50 Gew.% |

Dieses Schaumbad-Konzentrat war eine geschmeidige, stabile, cremige Zubereitung. Bei bestimmungsgemäßer Anwendung zeigte sich sowohl an der Haut als auch in der Haarbehandlung ein weiches, spannungsfreies Hautempfinden.

## Patentansprüche

1. Wasser/Öl-Misch-Emulgator mit einem Gehalt an
A. einer Mischung aus C-10 bis C-28 n-Fettalkoholen der folgenden Zusammensetzung:
| | |
|---|---|
| C-10: | 0,1 bis 1,0 Gew.% |
| C-12: | 1,0 bis 3,0 Gew.% |
| C-14: | 12,0 bis 16,0 Gew.% |
| C-16: | 16,0 bis 21,0 Gew.% |
| C-18: | 11,0 bis 14,5 Gew.% |
| C-20: | 6,0 bis 10,0 Gew.% |
| C-22: | 7,0 bis 11,0 Gew.% |
| C-24: | 2,5 bis 5,0 Gew.% |
| C-26: | 0,5 bis 2,5 Gew.% |
| C-28: | 0,5 bis 1,1 Gew.% |
B. einer Mischung aus C-20 bis C-24 2-Alkylalkanolen in einer Menge von 5 bis 23 Gew.% und
C. Phytosterolen in einer Menge von 6 bis 13 Gew.%.

2. Wasser/Öl-Misch-Emulgator nach Anspruch 1, **dadurch gekennzeichnet,** daß die Phytosterole (a) im wesentlichen
50 bis 85 Gew.% Sitosterin
6 bis 40 Gew.% Campesterin
0,1 bis 10 Gew.% Stigmasterin
enthalten.

3. Wasser/Öl-Misch-Emulgator nach einem der Ansprüche 1 oder 2, **dadurch gekennzeichnet,** daß die C-20 bis C-24 2-Alkylalkanole bezogen auf den Mischemulgator
2,0 bis 3,5 Gew.% C-20-2-Alkylalkanol
15,0 bis 19,0 Gew.% C-24-2-Alkylalkanol
enthalten.

4. Wasser/Öl-Misch-Emulgator nach Anspruch 3, **dadurch gekennzeichnet,** daß der Anteil der 2-Alkylalkanole in einem Bereich von
2,8 bis 3,0 Gew.% C-20-2-Alkylalkanol
16,9 bis 18,0 Gew.% C-24-2-Alkylalkanol
vorliegt.

5. Wasser/Öl-Misch-Emulgator nach einem der Ansprüche 1 bis 4, **dadurch gekennzeichnet,** daß die Mischung aus C-10 bis C-28 n-Fettalkoholen die folgende Zusammensetzung hat:
| | |
|---|---|
| C-10: | <1,0 Gew.% |
| C-12: | 1,2 bis 2,1 Gew.% |
| C-14: | 13,4 bis 15,4 Gew.% |
| C-16: | 17,7 bis 19,5 Gew.% |
| C-18: | 12,9 bis 13,8 Gew.% |
| C-20: | 7,6 bis 9,0 Gew.% |
| C-22: | 8,8 bis 10,5 Gew.% |
| C-24: | 2,9 bis 4,3 Gew.% |
| C-26: | 1,0 bis 2,0 Gew.% |
| C-28: | 0,6 bis 1,0 Gew.% |

6. Verwendung eines Wasser/Öl-Misch-Emulgators nach Anspruch 1 bis 5 in kosmetischen und pharmazeutischen Zubereitungen wie Salben, Cremes, Lotionen, Seifen, Shampoos und Badezusätzen.
